# FASCICULE DE BREVET EUROPEEN

(11) **EP 4 291 272 B1**
(45) Date de publication et mention de la délivrance du brevet: **29.01.2025**
(21) Numéro de dépôt: 22708993.5
(22) Date de dépôt: 14.02.2022
(51) Int. Cl.: A61M 11/00, A61M 15/00, A61M 15/08, B05B 11/02

(54) **TÊTE DE DISTRIBUTION POUR DISPOSITIF DE DISTRIBUTION NASALE D'UN PRODUIT FLUIDE OU PULVÉRULENT**
AUSGABEKOPF FÜR EINE VORRICHTUNG ZUR AUSGABE EINES FLÜSSIGEN ODER PULVERIGEN PRODUKTS IN DIE NASE
DISPENSING HEAD FOR A DEVICE FOR DISPENSING A FLUID OR POWDER PRODUCT INTO THE NOSE

(30) Priorité: 15.02.2021 FR 2101439
(43) Date de publication de la demande: 20.12.2023
(73) Titulaire: APTAR France SAS, 27110 Le Neubourg (FR)
(72) Inventeur: NORRANT, Matthieu, 27400 Heudebouville (FR); GRAINE, Lila, 78650 Beynes (FR)
(74) Mandataire: CAPRI
(86) Numéro de dépôt international: PCT/FR2022/050266
(87) Numéro de publication internationale: WO 2022/171969

(56) Documents cités:
- EP-B1- 2 976 121
- WO-A1-98/57690
- FR-A1- 2 739 294
- JP-A- 2012 135 535
- JP-A- H09 140 797

## Description

La présente invention concerne une tête de distribution pour dispositif de distribution nasale d'un produit fluide ou pulvérulent.

Les dispositifs de distribution nasale sont largement répandus et utilisés dans des applications diverses. Ces dispositifs comportent une tête de distribution incorporant l'orifice de distribution du produit, au moins une partie de ladite tête étant destinée à être insérée dans la narine de l'utilisateur lors de la distribution du produit. L'utilisateur actionne ensuite le dispositif pour expulser une dose de produit dans la narine.

Dans certaines applications, notamment dans le domaine de la pharmacie, les produits distribués par les dispositifs de distribution nasale peuvent être coûteux et/ ou doivent être dosés de manière très précise. On recherche par conséquent à obtenir une efficacité maximale de la dose de produit distribué dans la narine à chaque actionnement.

Or, la profondeur d'insertion de la tête de distribution dans la narine a un effet sur la distribution de la dose. Ainsi, une insertion trop profonde peut non seulement s'avérer douloureuse, mais également altérer la bonne distribution de la dose.

Pour remédier à ce problème, il a été proposé un embout qui se fixe sur la tête de distribution et qui permet non seulement de limiter la profondeur d'insertion dans la narine, mais aussi d'orienter dans une certaine mesure la tête de distribution dans la narine pour optimiser la distribution de la dose. Les documents WO9857690 et WO0027430 décrivent de tels embouts.

Or, selon l'utilisateur, la dimension de la narine peut varier assez fortement, notamment en raison de nombreux paramètres, tels que le sexe, l'âge, la taille ou l'origine ethnique de l'utilisateur.

Un inconvénient des embouts des documents WO9857690 et WO0027430 réside dans le fait qu'ils ne permettent pas de tenir compte des différentes dimensions des narines, et que pour pallier à cet inconvénient, il faut prévoir une pluralité d'embouts amovibles de tailles différentes, l'utilisateur choisissant la taille qui lui convient avant utilisation. Cette mise en oeuvre oblige de fabriquer de très nombreux embouts, par exemple trois ou quatre tailles pour chaque dispositif, alors qu'un seul de ces embouts sera en réalité utilisé. Ceci génère un surcoût de fabrication important, augmente les dimensions du packaging du dispositif, consomme plus de matière plastique et risque donc d'augmenter la pollution environnementale.

Les documents JP2012135535, JPH09140797, FR2739294 et EP2976121 décrivent d'autres dispositifs de l'état de la technique.

C'est un but de la présente invention que de fournir une tête de distribution nasale qui ne reproduit pas les inconvénients précités.

Plus particulièrement, c'est un but de la présente invention que de fournir une tête de distribution nasale qui permet, lors de l'actionnement du dispositif, de définir la bonne profondeur d'insertion du dispositif dans la narine adaptée pour chaque utilisateur.

C'est également un but de la présente invention que de fournir une tête de distribution nasale du type précité qui soit simple et peu coûteuse à fabriquer et à assembler.

C'est également un but de la présente invention que de fournir une telle tête de distribution nasale qui soit d'utilisation simple et fiable pour n'importe quel utilisateur.

La présente invention a donc pour objet une tête de distribution nasale d'un produit fluide ou pulvérulent, comportant une partie d'extrémité axiale pourvue d'un orifice de distribution, ladite partie d'extrémité axiale de ladite tête étant destinée à être insérée dans une narine lors de l'actionnement, ladite tête comprenant un embout adapté, lors de l'actionnement, à s'appuyer sur l'entrée de la narine pour définir la profondeur d'insertion de ladite tête dans la narine, ledit embout étant déplaçable axialement sur ladite tête entre au moins deux positions d'utilisations décalées axialement l'une de l'autre, ledit embout comportant un manchon axial cylindrique creux disposé autour d'une partie cylindrique de ladite tête, ledit manchon se prolongeant du côté axial supérieur par une bride radiale qui s'étend radialement vers l'extérieur, ladite bride radiale s'appuyant sur le nez autour de l'entrée de la narine lors de l'actionnement, ladite partie cylindrique comportant au moins deux évidements radiaux externes décalés axialement, et au moins un méplat s'étendant axialement dans ladite partie cylindrique, chaque méplat comportant au moins deux nervures s'étendant radialement vers l'extérieur, chaque nervure étant disposée au niveau d'un évidement radial externe respectif, ledit manchon comportant au moins une projection radiale interne, le diamètre interne dudit manchon au niveau de ladite au moins une projection radiale interne étant supérieur au diamètre externe de la partie cylindrique au niveau dudit au moins un méplat, environ identique au diamètre externe de la partie cylindrique au niveau de chaque nervure et inférieur au diamètre externe de la partie cylindrique au niveau de chaque évidement radial externe, de sorte que ledit embout peut coulisser axialement sur ladite tête lorsque ladite au moins une projection radiale interne est disposée au niveau d'un méplat et ledit embout ne peut pas coulisser axialement sur ladite tête lorsque ladite au moins une projection radiale interne est disposée dans un évidement radial externe, après rotation dudit embout sur ladite tête, ladite au moins une projection radiale interne étant disposée dans un premier évidement radial dans une première position d'utilisation et dans un second évidement radial dans une seconde position d'utilisation.

Avantageusement, ladite partie cylindrique comporte deux méplats diamétralement opposés, trois évidements radiaux externes et trois nervures, chaque évidement radial externe s'étendant sur la périphérie de ladite partie cylindrique en étant interrompu au niveau de chaque méplat, et chaque nervure s'étendant sur chacun desdits deux méplats.

Avantageusement, ledit manchon comporte deux projections radiales internes diamétralement opposées.

Avantageusement, ladite bride radiale a un bord radialement externe de forme arrondie, notamment circulaire ou ovale.

La présente invention a aussi pour objet un dispositif de distribution de produit fluide, comportant un réservoir et un organe de distribution, tel qu'un piston, monté coulissant dans ledit réservoir, ledit dispositif comportant une tête de distribution telle que décrite ci-dessus.

Avantageusement, ledit réservoir contient une seule dose de produit fluide distribuée en un seul actionnement.

Avantageusement, ledit réservoir contient deux doses de produit fluide distribuées en deux actionnements successifs.

Ces caractéristiques et avantages et d'autres de la présente invention apparaîtront plus clairement dans la description détaillée suivante donnée à titre d'exemple non limitatif en référence aux dessins joints, sur lesquels :
la figure 1 est une vue schématique en section transversale d'un dispositif de distribution nasale de produit fluide en position de repos,
les figures 2 et 3 sont des vues schématiques de détail en section transversale, montrant l'embout selon un premier mode de réalisation, dans deux positions d'utilisations différentes,
les figures 4 à 6 sont des vues schématiques de détail en section transversale, montrant l'embout selon un second mode de réalisation, dans trois positions d'utilisations différentes,
les figures 7 à 9 sont des vues schématiques de détail de côté, montrant l'embout selon un troisième mode de réalisation, dans trois positions d'utilisations différentes,
les figures 10 à 12 sont des vues schématiques de détail en section transversale, montrant l'embout du troisième mode de réalisation dans ses trois positions d'utilisations différentes,
la figure 13 est une vue schématique de détail en perspective, montrant la tête de distribution selon un quatrième mode de réalisation,
la figure 14 est une vue schématique de détail en perspective, montrant l'embout du quatrième mode de réalisation,
la figure 15 est une vue schématique de détail en perspective, montrant l'embout du quatrième mode de réalisation coopérant avec la tête de distribution, et
les figures 16 à 18 sont des vues schématiques de détail en section transversale, montrant l'embout du quatrième mode de réalisation dans trois positions d'utilisations différentes. Les figures 1 à 12 concernent des modes de réalisation utiles à la compréhension de l'invention mais non couvert par les revendications.

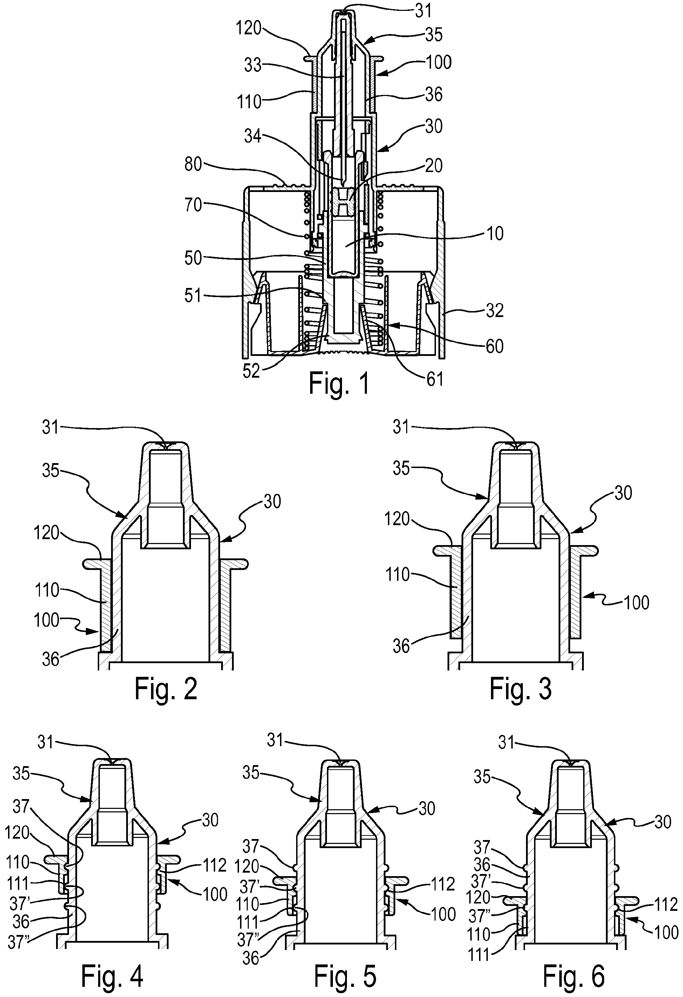

Dans la description ci-après, les termes "axial" et "radial" se réfèrent à l'axe central longitudinal du dispositif. Les termes "supérieur", "inférieur", "haut" et "bas" se réfèrent à la position droite du dispositif représentée sur la figure 1.

La présente invention sera décrite ci-après en référence à un exemple de réalisation qui est un bidose, c'est-à-dire un dispositif contenant deux doses de produit fluide à distribuer lors de deux actionnements successifs du dispositif. Il est toutefois entendu que la présente invention pourrait s'appliquer à des dispositifs comportant une seule dose ou un nombre supérieur de doses, par exemple trois ou quatre doses. De même, le dispositif de type bidose représenté sur les dessins n'est qu'un exemple de réalisation possible auquel la présente invention s'applique, et il est entendu que la présente invention s'applique de manière plus générale à tout type de dispositif contenant au moins une dose.

En référence à la figure 1, le dispositif de distribution nasale comporte un réservoir 10 contenant deux doses de produit fluide. Un organe de distribution, tel qu'un piston 20, est monté coulissant dans ledit réservoir 10. Dans la position avant actionnement du dispositif, représentée sur la figure 1, ledit piston 20 fait office de bouchon en isolant le contenu du réservoir 10.

Une tête de distribution 30 est assemblée sur ledit réservoir 10 en étant déplaçable axialement par rapport à celui-ci. En particulier, un déplacement axial de la tête de distribution 30 par rapport au réservoir 10 provoque le déplacement du piston 20 dans le réservoir 10 et ainsi la distribution du produit fluide contenu dans ledit réservoir. La tête de distribution 30 comporte un canal de distribution 33 qui mène depuis une pointe de percement 34 jusqu'à un orifice de distribution 31 de la tête de distribution 30. En amont de l'orifice de distribution 31, il peut être prévu un profil de pulvérisation, qui peut être d'un quelconque type connu et qui n'est pas représenté plus en détail sur le dessin, pour distribuer le produit fluide sous forme de spray.

Plus précisément, dans l'exemple représenté, le réservoir 10 est fixé dans un corps 50, qui est donc solidaire dudit réservoir 10, et qui se déplace ensemble avec lui.

La tête de distribution 30 comporte une jupe latérale inférieure 32 adaptée à coopérer avec un organe d'actionnement 60. Un élément repose-doigts 80 est formé de manière monobloc avec ladite tête de distribution 30, ou en variante est assemblé autour d'elle.

Ledit organe d'actionnement 60 est axialement déplaçable à l'intérieur de ladite jupe latérale 32 de la tête de distribution 30 pour réaliser les actionnements successifs de dispositif. L'organe d'actionnement 60 comporte au moins une patte inclinée 61 qui est adaptée à coopérer avec des projections 51, 52 du corps 50 pour réaliser les actionnements successifs.

Un ressort de rappel 70 est monté entre l'organe d'actionnement 60 et la tête de distribution 30, pour ramener ledit organe d'actionnement 60 dans sa position de départ après chaque actionnement.

Le fonctionnement du dispositif représenté sur la figure 1 est le suivant. Dans la position de repos de la figure 1, le piston bouchon 20 isole le contenu du réservoir 10 de l'atmosphère. Lorsque l'utilisateur appuie simultanément sur le repose-doigt 80 et sur l'organe d'actionnement 60, il va déplacer ledit organe d'actionnement 60 à l'intérieur de la jupe latérale 32 de la tête de distribution 30. Ceci va pousser le corps 50 axialement vers le haut, par l'intermédiaire des pattes 61 qui vont pousser sur l'épaulement 51 dudit corps. Ceci va comprimer le ressort 70 et déplacer le réservoir 10 par rapport à la tête de distribution 30. Lorsque le réservoir 10 va commencer à se déplacer par rapport à la tête de distribution 30, l'extrémité de percement 34 du canal de distribution 33 va venir percer le piston de bouchon 20 pour faire communiquer l'intérieur du réservoir 10 avec ledit canal d'expulsion 33. Une poursuite de l'actionnement va provoquer un déplacement du piston 20 à l'intérieur du réservoir et donc une distribution de la première dose. Le produit fluide est donc poussé par ledit piston 20 à travers l'extrémité de percement 34 dans le canal de distribution 33, puis hors du dispositif à travers l'orifice de distribution 31.

Après distribution de la première dose, lorsque l'utilisateur relâche l'organe d'actionnement 60, le ressort 70 va ramener celui-ci vers sa position de départ. Pendant ce retour de l'organe d'actionnement 60, le réservoir et le corps 50 ne reviennent pas en arrière, car ces deux composants restent maintenus dans ladite tête de distribution 30. Eventuellement, la tête de distribution 30 peut comporter des moyens anti-retour pour empêcher un retour dudit corps 50 et/ou dudit réservoir 10 en arrière. Lorsque l'organe d'actionnement 60 revient sous l'effet du ressort de rappel 70 vers sa position de repos, les pattes 61 vont se positionner sous la deuxième projection 52 du corps 50 ce qui va permettre à l'utilisateur d'actionner une deuxième fois le dispositif pour distribuer la deuxième dose de produit fluide.

Le dispositif de distribution peut comporter un indicateur (non représenté) pour indiquer à l'utilisateur la distribution de la première dose et la distribution de la deuxième dose. De cette manière, l'utilisateur sait exactement où il en est et si la première dose a ou non été distribuée. Cet indicateur peut comporter une fenêtre de visualisation formée dans ladite tête de distribution 30.

Le dispositif de distribution nasale comporte un embout 100 qui est disposé sur la tête 30 pour définir la profondeur d'insertion de la tête de distribution dans la narine.

Avantageusement, l'embout 100 comporte un manchon axial cylindrique creux 110 disposé autour d'une partie cylindrique 36 de ladite tête 30, ledit manchon axial 110 se terminant du côté axial supérieur par une bride radiale 120 qui s'étend radialement vers l'extérieur. Cette bride radiale 120 de l'embout 100 vient s'appuyer sur le nez, autour de la narine, pour définir la profondeur d'insertion de la tête 30 dans la narine.

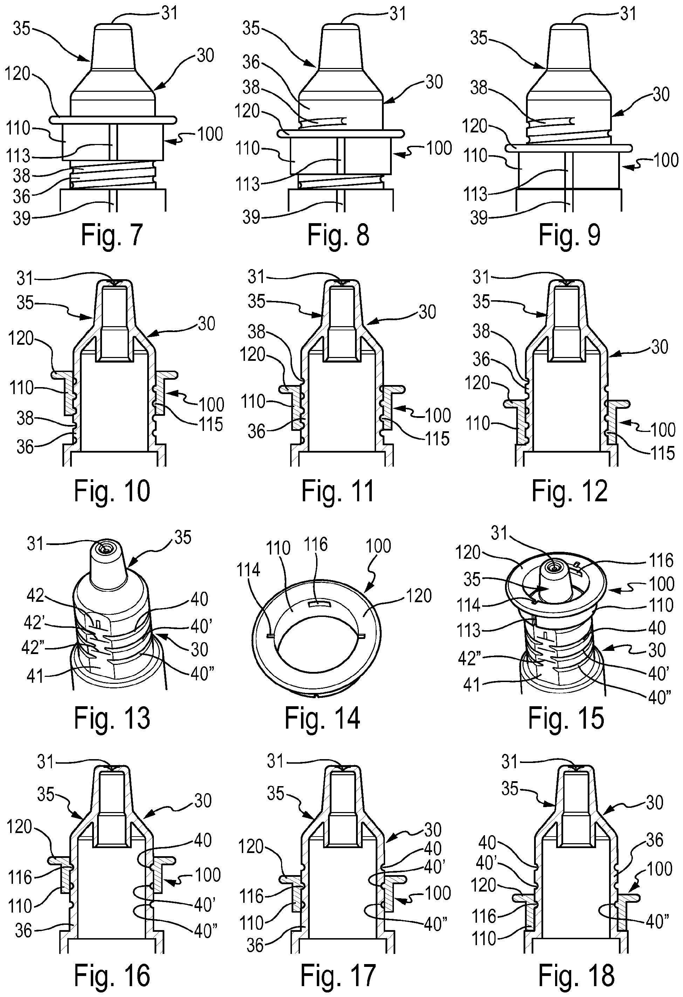

Le bord radialement externe de la bride radiale 120 est avantageusement de forme arrondie, par exemple circulaire comme représenté sur les figures 14 et 15, mais toute autre forme est envisageable, par exemple une forme ovale.

De par sa dimension radiale, la bride radiale 120 permet aussi d'orienter dans une certaine mesure la tête 30 dans la narine, avec notamment le bord radialement externe de ladite bride radiale 120 qui peut venir s'appuyer sur la lèvre supérieure, sous la narine, comme décrit dans le document WO9857690.

Selon l'invention, l'embout 100 est ajustable avant chaque utilisation du dispositif, c'est-à-dire qu'il est déplaçable axialement sur la tête 30 entre au moins deux positions d'utilisations décalées axialement l'une de l'autre. Les figures montrent trois modes de réalisation différents, avec deux ou trois positions d'utilisation différentes, mais il est entendu qu'un nombre quelconque de positions différentes est envisageable.

Les figures 2 et 3 illustrent un premier mode de réalisation.

Dans ce premier mode de réalisation, le manchon 110 de l'embout 100 est emmanché à force sur la partie cylindrique 36 de ladite tête 30.

La figure 2 montre une première position d'utilisation de l'embout, dans laquelle l'embout 100 est dans une position inférieure, avec donc une plus grande partie de la tête qui pénètrera dans la narine lors de l'actionnement.

La figure 3 montre une seconde position d'utilisation de l'embout, dans laquelle l'embout 100 est dans une position supérieure, décalée axialement vers le haut par rapport à la première position inférieure de la figure 2, avec donc une plus petite partie de la tête qui pénètrera dans la narine lors de l'actionnement.

La force nécessaire pour déplacer axialement l'embout 100 sur la tête 30 entre ses positions d'utilisation est supérieure à la force d'appui dudit embout 100 sur le nez lors de l'actionnement. Ainsi, il n'y a pas de risque que l'embout se déplace sur la tête lors de l'actionnement.

Les figures 4 à 6 illustrent un second mode de réalisation.

Dans ce second mode de réalisation, la partie cylindrique 36 comporte au moins deux projections radiales externes 37; 37'; 37" décalées axialement et le manchon 110 comporte au moins un évidement radial interne 112.

L'évidement radial interne 112 coopère, notamment par encliquetage, avec une première projection radiale 37 dans une première position d'utilisation, qui est une position d'utilisation supérieure, représentée sur la figure 4.

Dans une seconde position d'utilisation intermédiaire, visible sur la figure 5, l'évidement radial interne 112 coopère avec une seconde projection radiale 37', et dans une troisième position d'utilisation, qui est une position d'utilisation inférieure visible sur la figure 6, il coopère avec une troisième projection radiale 37".

En variante, ou pourrait remplacer les projections radiales de la tête par des évidements, et l'évidement de l'embout par une projection.

Avantageusement, l'embout 100 comporte un évidement radial interne 112 périphérique. Ceci permet de ne pas avoir à orienter l'embout angulairement par rapport à la tête.

Dans l'exemple représenté, l'évidement radial 112 est disposé à proximité de la bride radiale 120, et la manchon 110 comporte alors avantageusement une découpe 111, de préférence périphérique, sur sa partie inférieure pour recevoir la ou les projection(s) radiale(s) disposée(s) en-dessous de la projection radiale qui coopère avec l'évidement radial 112.

Avantageusement, chaque projection radiale externe 37; 37'; 37" est périphérique, formant un bourrelet tout autour de la tête. En variante, on pourrait avoir un ou plusieurs ergots saillants au lieu d'un bourrelet périphérique.

Les figures 7 à 12 illustrent un troisième mode de réalisation.

Dans ce troisième mode de réalisation, la partie cylindrique 36 comporte un filetage externe 38 et le manchon 110 comporte un filetage interne 115 coopérant avec ledit filetage externe 38, l'embout 100 étant déplaçable en rotation sur la tête 30 entre ses positions d'utilisation.

Avantageusement, le frottement entre lesdits filetages interne et externe 115, 38 empêche une rotation dudit embout 100 lors de l'actionnement. En d'autres termes, il n'y a pas de risque que l'embout se déplace sur la tête lors de l'actionnement.

Dans l'exemple représenté sur les figures, le filetage externe 38 est un filetage femelle, c'est-à-dire que le filet est creusé dans la partie cylindrique 36 et le filetage interne 115 est un filetage male, c'est-à-dire que le filet se projette radialement vers l'intérieur à partir dudit manchon 110. Bien entendu, la mise en oeuvre inverse est aussi possible.

Avantageusement, on définit trois positions d'utilisation, à savoir une position supérieure, représentée sur les figures 7 et 10, une position intermédiaire, représentée sur les figures 8 et 11, et une position inférieure, représentée sur les figures 9 et 12. On peut alors avantageusement prévoir un repère 113 sur l'extérieur du manchon 110 de l'embout 100, et un repère correspondant 39 sur la tête 30, notamment sur une partie de la tête 30 disposée en-dessous de l'embout 100. Comme visible sur les figures 7 à 9, la correspondance du repère 113 de l'embout avec le repère 39 de la tête définit la position d'utilisation respective. Eventuellement, on peut prévoir des points de résistance sur le filetage externe 38 et/ou le filetage interne 115 (non représentés) pour définir ces différentes positions d'utilisation de l'embout.

Les figures 13 à 18 illustrent un quatrième mode de réalisation selon l'invention.

Dans ce quatrième mode de réalisation, la partie cylindrique 36 comporte au moins deux évidements radiaux externes 40; 40'; 40" décalés axialement.

Au moins un méplat 41 s'étend axialement dans ladite partie cylindrique 36. Avantageusement, on prévoit deux méplats diamétralement opposés.

Chaque méplat 41 comporte au moins deux nervures 42; 42'; 42" s'étendant radialement vers l'extérieur, chaque nervure 42; 42'; 42" étant disposée au niveau d'un évidement radial externe 40; 40'; 40" respectif. Avantageusement, on prévoit trois évidements radiaux externes 40; 40'; 40" et trois nervures 42; 42'; 42", chaque évidement radial externe 40; 40'; 40" s'étendant sur la périphérie de la partie cylindrique 36 en étant interrompu au niveau de chaque méplat 41, et chaque nervure 42; 42'; 42" s'étendant sur chacun des méplats 41.

Le manchon 110 comporte au moins une projection radiale interne 116. Avantageusement, le manchon 110 comporte deux projections radiales internes 116 diamétralement opposées.

Le diamètre interne dudit manchon 110 au niveau des projections radiales internes 116 est supérieur au diamètre externe de la partie cylindrique 36 au niveau dudit au moins un méplat 41, environ identique au diamètre externe de la partie cylindrique 36 au niveau de chaque nervure 42; 42'; 42", et inférieur au diamètre externe de la partie cylindrique 36 au niveau de chaque évidement radial externe 40; 40'; 40". Ainsi, l'embout 100 peut coulisser axialement sur la tête 30 lorsque chaque projection radiale interne 116 est disposée au niveau d'un méplat 41 respectif et l'embout 100 ne peut pas coulisser axialement sur la tête 30 lorsque chaque projection radiale interne 116 est disposée dans un évidement radial externe 40; 40'; 40" respectif, après rotation de l'embout 100 sur la tête 30.

Dans l'exemple représenté, où la tête comprend trois évidements radiaux externes 40; 40'; 40" et trois nervures 42; 42'; 42", on définit trois positions d'utilisation, à savoir une position supérieure, représentée sur la figure 16, une position intermédiaire, représentée sur la figure 17, et une position inférieure, représentée sur la figure 18.

Dans la position d'utilisation supérieure, chaque projection radiale interne 116 est disposée dans un premier évidement radial 40 respectif. Dans la position d'utilisation intermédiaire, chaque projection radiale interne 116 est disposée dans un second évidement radial 40' respectif. Dans la position d'utilisation inférieure, chaque projection radiale interne 116 est disposée dans un troisième évidement radial 40" respectif.

Les nervures 42; 42'; 42" servent à identifier aisément chacune des position axiales de l'embout. En effet, de par leur surépaisseur par rapport au méplat 41, elles coopèrent avec la ou les projection(s) radiale(s) interne(s) 116 pour former une résistance au coulissement axial de l'embout 100. Ainsi, ces nervures n'empêchent pas ce coulissement, mais chaque nervure permet d'identifier avec précision la position axiale de chaque évidement radial 40, 40', 40" respectif. Ainsi, l'utilisateur qui veut passer de la position supérieure à la position intermédiaire, tourne l'embout 100 de telle sorte que chaque projection radiale interne 116 se trouve face à un méplat 41 respectif et appuie sur l'embout pour le déplacer axialement vers le bas. Lorsque la projection radiale interne atteint la nervure 42' de la position intermédiaire, le déplacement axial de l'embout est stoppé, et l'utilisateur peut alors tourner l'embout par rapport à la tête pour amener chaque projection radiale 116 dans un évidement radial 40' respectif. Si par contre l'utilisateur veut aller à la position inférieure, il appui plus fort sur l'embout pour surmonter la résistance générée par la nervure 42', et il peut alors déplacer axialement l'embout davantage vers le bas.

Avantageusement, l'embout 100 comporte un repère 113 dur le manchon 110, disposé au niveau de chaque projection radiale interne 116. Eventuellement, on peut également prévoir un second repère 114 sur la bride radiale 120, également disposé au niveau de chaque projection radiale interne 116. Ceci permet de guider l'utilisateur pour identifier aisément la position angulaire dans laquelle il peut déplacer l'embout axialement par rapport à la tête, en alignant le ou les repères 113, 114 avec un méplat 41.

D'autres variantes de réalisation sont imaginables pour réaliser la fonction revendiquée par la présente invention.

Le fonctionnement du dispositif est le suivant.

Avant utilisation, l'utilisateur règle la position d'utilisation de l'embout 100 sur l'extrémité axiale de la tête 30. Il insère alors la tête 30, du moins son extrémité axiale, dans sa narine. Pendant cette opération, la bride radiale 120 de l'embout 100 vient s'appuyer sur l'aile du nez, pour définir la profondeur d'insertion de ladite tête 30 dans la narine. L'utilisateur actionne alors le dispositif et la dose de produit est distribuée dans la narine.

La présente invention permet donc de fournir une tête de distribution nasale simple et peu coûteuse à fabriquer (notamment à mouler) et à assembler. Cette tête est en outre simple et fiable à utiliser par tout le monde. Ceci est particulièrement avantageux dans le domaine de la pharmacie où le dosage du produit doit être très précis et son efficacité optimisée en raison du coût souvent élevé des produits à distribuer.

La présente invention a été décrite en référence à plusieurs modes de réalisation particuliers de celle-ci, mais il est clair que diverses modifications sont possibles sans sortir du cadre de la présente invention tel que défini par les revendications annexées.

## Revendications

1. Tête de distribution nasale (30) d'un produit fluide ou pulvérulent, comportant une partie d'extrémité axiale (35) pourvue d'un orifice de distribution (31), ladite partie d'extrémité axiale (35) de ladite tête (30) étant destinée à être insérée dans une narine lors de l'actionnement, ladite tête comprenant un embout (100) adapté, lors de l'actionnement, à s'appuyer sur l'entrée de la narine pour définir la profondeur d'insertion de ladite tête (30) dans la narine, ledit embout (100) étant déplaçable axialement sur ladite tête (30) entre au moins deux positions d'utilisations décalées axialement l'une de l'autre, ledit embout (100) comportant un manchon axial cylindrique creux (110) disposé autour d'une partie cylindrique (36) de ladite tête (30), ledit manchon (110) se prolongeant du côté axial supérieur par une bride radiale (120) qui s'étend radialement vers l'extérieur, ladite bride radiale (120) s'appuyant sur le nez autour de l'entrée de la narine lors de l'actionnement, **caractérisée en ce que** ladite partie cylindrique (36) comporte au moins deux évidements radiaux externes (40; 40'; 40") décalés axialement, et au moins un méplat (41) s'étendant axialement dans ladite partie cylindrique (36), chaque méplat (41) comportant au moins deux nervures (42; 42'; 42") s'étendant radialement vers l'extérieur, chaque nervure (42; 42'; 42") étant disposée au niveau d'un évidement radial externe (40; 40'; 40") respectif, ledit manchon (110) comportant au moins une projection radiale interne (116), le diamètre interne dudit manchon (110) au niveau de ladite au moins une projection radiale interne (116) étant supérieur au diamètre externe de la partie cylindrique (36) au niveau dudit au moins un méplat (41), environ identique au diamètre externe de la partie cylindrique (36) au niveau de chaque nervure (42; 42'; 42") et inférieur au diamètre externe de la partie cylindrique (36) au niveau de chaque évidement radial externe (40; 40'; 40"), de sorte que ledit embout (100) peut coulisser axialement sur ladite tête (30) lorsque ladite au moins une projection radiale interne (116) est disposée au niveau d'un méplat (41) et ledit embout (100) ne peut pas coulisser axialement sur ladite tête (30) lorsque ladite au moins une projection radiale interne (116) est disposée dans un évidement radial externe (40; 40'; 40"), après rotation dudit embout (100) sur ladite tête (30), ladite au moins une projection radiale interne (116) étant disposée dans un premier évidement radial (40) dans une première position d'utilisation et dans un second évidement radial (40') dans une seconde position d'utilisation.

2. Tête selon la revendication 1, dans laquelle ladite partie cylindrique (36) comporte deux méplats (41) diamétralement opposés, trois évidements radiaux externes (40; 40'; 40") et trois nervures (42; 42'; 42"), chaque évidement radial externe (40; 40'; 40") s'étendant sur la périphérie de ladite partie cylindrique (36) en étant interrompu au niveau de chaque méplat (41), et chaque nervure (42; 42'; 42") s'étendant sur chacun desdits deux méplats (41).

3. Tête selon la revendication 1 ou 2, dans laquelle ledit manchon (110) comporte deux projections radiales internes (116) diamétralement opposées.

4. Tête selon l'une quelconque des revendications précédentes, dans laquelle ladite bride radiale (120) a un bord radialement externe de forme arrondie, notamment circulaire ou ovale.

5. Dispositif de distribution de produit fluide, comportant un réservoir (10) et un organe de distribution (20), tel qu'un piston, monté coulissant dans ledit réservoir (10), **caractérisé en ce que** ledit dispositif comporte une tête de distribution (30) selon l'une quelconque des revendications précédentes.

6. Dispositif selon la revendication 5, dans lequel ledit réservoir (10) contient une seule dose de produit fluide distribuée en un seul actionnement.

7. Dispositif selon la revendication 5, dans lequel ledit réservoir (10) contient deux doses de produit fluide distribuées en deux actionnements successifs.

## Patentansprüche

1. Kopf (30) zur intranasalen Verabreichung eines flüssigen oder pulverförmigen Produkts, mit einem axialen Endabschnitt (35), der mit einer Abgabeöffnung (31) versehen ist, wobei der axiale Endabschnitt (35) des Kopfes (30) zur Einführung in ein Nasenloch bei Betätigung bestimmt ist, der Kopf ein Ansatzstück (100) umfasst, das derart ausgelegt ist, dass es bei Betätigung am Eingang des Nasenlochs anliegt, um die Einführtiefe des Kopfes (30) in das Nasenloch zu definieren, wobei das Ansatzstück (100) auf dem Kopf (30) zwischen mindestens zwei axial gegeneinander versetzten Gebrauchsstellungen axial verschiebbar ist, das Ansatzstück (100) eine hohlzylindrische axiale Hülse (110) aufweist, die um einen zylindrischen Teil (36) des Kopfes (30) herum angeordnet ist, wobei die Hülse (110) an der oberen axialen Seite durch einen Radialflansch (120) verlängert ist, der radial nach außen verläuft, wobei bei Betätigung der Radialflansch (120) um den Nasenlocheingang herum an der Nase anliegt, **dadurch gekennzeichnet, dass** der zylindrische Teil (36) mindestens zwei äußere radiale Ausnehmungen (40, 40', 40") aufweist, die axial versetzt sind, und mindestens eine Abflachung (41), die sich axial in den zylindrischen Teil (36) erstreckt, wobei jede Abflachung (41) mindestens zwei Rippen (42; 42'; 42") aufweist, die sich radial nach außen erstrecken, wobei jede Rippe (42; 42'; 42") an einer radial äußeren Ausnehmung (40; 40'; 40") angeordnet ist, wobei die Hülse (110) mindestens einen inneren radialen Vorsprung (116) aufweist, wobei der Innendurchmesser der Hülse (110) an dem mindestens einen inneren radialen Vorsprung (116) größer ist als der Außendurchmesser des zylindrischen Teils (36) an der mindestens einen Abflachung (41), etwa gleich dem Außendurchmesser des zylindrischen Abschnitts (36) an jeder Rippe (42; 42'; 42"), und kleiner als der Außendurchmesser des zylindrischen Abschnitts (36) an jeder äußeren radialen Aussparung (40; 42'; 42"), sodass ein axiales Gleiten des Ansatzstücks (100) auf dem Kopf (30) möglich ist, wenn der mindestens eine innere radiale Vorsprung (116) an einer Abflachung (41) angeordnet ist, und ein axiales Gleiten des Ansatzstücks (100) auf dem Kopf (30) nicht möglich ist, wenn der mindestens eine innere radiale Vorsprung (116) in einer äußeren radialen Ausnehmung (40, 40'; 40") angeordnet ist, nachdem das Ansatzstück (100) auf dem Kopf (30) gedreht wurde, wobei in einer ersten Gebrauchsstellung der mindestens eine innere radiale Vorsprung (116) in einer ersten radialen Aussparung (40) angeordnet ist und in einer zweiten Gebrauchsstellung dieser in einer zweiten radialen Aussparung (40') angeordnet ist.

2. Kopf nach Anspruch 1, bei dem der zylindrische Teil (36) zwei diametral gegenüberliegende Abflachungen (41), drei äußere radiale Aussparungen (40; 40'; 40") und drei Rippen (42; 42'; 42") aufweist, wobei jede äußere radiale Aussparung (40; 40'; 40") sich über den Umfang des zylindrischen Teils (36) erstreckt und an jeder Abflachung (41) unterbrochen ist, und jede Rippe (42; 42'; 42") sich über jede der beiden Abflachungen (41) erstreckt.

3. Kopf nach Anspruch 1 oder 2, bei dem die Hülse (110) zwei einander diametral gegenüberliegende innere radiale Vorsprünge (116) aufweist.

4. Kopf nach einem der vorhergehenden Ansprüche, bei dem der radiale Flansch (120) einen radial äußeren Rand mit einer abgerundeten Form, insbesondere einer kreisförmigen oder ovalen Form hat.

5. Vorrichtung zur Abgabe eines flüssigen Produkts, mit einem Vorratsbehälter (10) und einem Abgabeorgan (20), beispielsweise einem Kolben, das gleitend in dem Vorratsbehälter (10) installiert ist, **dadurch gekennzeichnet, dass** die Vorrichtung einen Abgabekopf (30) gemäß einem der vorhergehenden Ansprüche aufweist.

6. Vorrichtung nach Anspruch 5, bei der jeder Vorratsbehälter (10) eine Einzeldosis des flüssigen Produkts enthält, deren Abgabe durch eine einzige Betätigung erfolgt.

7. Vorrichtung nach Anspruch 5, bei der der Vorratsbehälter (10) zwei Dosen des flüssigen Produkts enthält, deren Abgabe durch zwei aufeinanderfolgende Betätigungen erfolgt.

## Claims

1. Head (30) for dispensing a fluid or powder product into the nose, comprising an axial end portion (35) provided with a dispensing opening (31), said axial end portion (35) of said head (30) being intended to be inserted into a nostril during actuation, said head comprising a tip (100) suitable for bearing on the nostril entrance during actuation, to define the depth to which said head (30) is inserted into the nostril, said tip (100) being axially movable on said head (30) between at least two positions of use axially offset from each other, said tip (100) comprising a hollow cylindrical axial sleeve (110) disposed around a cylindrical portion (36) of said head (30), said sleeve (110) extending from the upper axial side by a radial flange (120) which extends radially outwards, said radial flange (120) bearing on the nose around the nostril entrance during actuation, **characterised in that** said cylindrical portion (36) comprises at least two axially offset external radial recesses (40; 40'; 40"), and at least one flat spot (41) extending axially into said cylindrical portion (36), each flat spot (41) comprising at least two ribs (42; 42'; 42") extending radially outwards, each rib (42; 42'; 42") being disposed at a respective external radial recess (40; 40'; 40"), said sleeve (110) comprising at least one internal radial projection (116), the internal diameter of said sleeve (110) at said at least one internal radial projection (116) being greater than the external diameter of the cylindrical portion (36) at said at least one flat spot (41), almost identical to the external diameter of the cylindrical portion (36) at each rib (42; 42'; 42") and less than the external diameter of the cylindrical portion (36) at each external radial recess (40; 40'; 40"), such that said tip (100) can axially slide on said head (30) when said at least one internal radial projection (116) is disposed at a flat spot (41) and said tip (100) cannot axially slide on said head (30) when said at least one internal radial projection (116) is disposed in an external radial recess (40; 40'; 40"), after rotation of said tip (100) on said head (30), said at least one internal radial projection (116) being disposed in a first radial recess (40) in a first position of use and in a second radial recess (40') in a second position of use.

2. Head according to claim 1, wherein said cylindrical portion (36) comprises two diametrically opposite flat spots (41), three external radial recesses (40; 40'; 40") and three ribs (42; 42'; 42"), each external radial recess (40; 40'; 40") extending over the periphery of said cylindrical portion (36) by being interrupted at each flat spot (41), and each rib (42; 42'; 42") extending over each of said two flat spots (41).

3. Head according to claim 1 or 2, wherein said sleeve (110) comprises two diametrically opposite internal radial projections (116).

4. Head according to any one of the preceding claims, wherein said radial flange (120) has a radially external edge with a rounded-shape, in particular circular or oval.

5. Device for dispensing fluid product, comprising a reservoir (10) and a dispensing member (20), such as a piston, slidingly mounted in said reservoir (10), **characterised in that** said device comprises a dispensing head (30) according to any one of the preceding claims.

6. Device according to claim 5, wherein said reservoir (10) contains one single dose of fluid product dispensed in one single actuation.

7. Device according to claim 5, wherein said reservoir (10) contains two doses of fluid product dispensed in two successive actuations.
